# FASCICULE DE BREVET EUROPEEN

(11) **EP 1 346 226 B1**
(45) Date de publication et mention de la délivrance du brevet: **31.05.2006**
(21) Numéro de dépôt: 01969918.0
(22) Date de dépôt: 18.09.2001
(51) Int. Cl.: G01N 33/68, G01N 33/574

(54) **PROCEDE DE CRIBLAGE BASE SUR L'INTERACTION SIAH-NUMB**
SCREENING-VERFAHREN AUF BASIS DER SIAH-NUMB-WECHSELWIRKUNG
SCREENING METHOD BASED ON SIAH-NUMB INTERACTION

(30) Priorité: 26.12.2000 FR 0017029
(43) Date de publication de la demande: 24.09.2003
(73) Titulaire: Cerenis, 75011 Paris (FR)
(72) Inventeur: AMSON, Robert, F-75005 Paris (FR); TELERMAN, Adam, F-75007 Paris (FR); SUSINI, Laurent, F-95160 Montmorency (FR); OREN, Moshe, 76282 Rehovot (IL)
(74) Mandataire: Warcoin, Jacques
(86) Numéro de dépôt international: PCT/FR2001/002895
(87) Numéro de publication internationale: WO 2002/052273

(56) Documents cités:
- WO-A-97/22695
- FR-A- 2 782 085
- ROPERCH JEAN-PIERRE ET AL: "SIAH-1 promotes apoptosis and tumor suppression through a network involving the regulation of protein folding, unfolding, and trafficking: Identification of common effectors with p53 and p21Waf1." PROCEEDINGS OF THE NATIONAL ACADEMY OF SCIENCES OF THE UNITED STATES, vol. 96, no. 14, 6 juillet 1999 (1999-07-06), pages 8070-8073, XP002182373 July 6, 1999 ISSN: 0027-8424
- JUVEN-GERSHON TAMAR ET AL: "The Mdm2 oncoprotein interacts with the cell fate regulator numb." MOLECULAR AND CELLULAR BIOLOGY, vol. 18, no. 7, juillet 1998 (1998-07), pages 3974-3982, XP001031399 ISSN: 0270-7306
- GERMANI ANTONIA ET AL: "SIAH-1 interacts with alpha-tubulin and degrades the kinesin Kid by the proteasome pathway during mitosis." ONCOGENE, vol. 19, no. 52, 2000, pages 5997-6006, XP001031384 ISSN: 0950-9232
- MATSUZAWA SHU-ICHI ET AL: "p53-inducible human homologue of Drosophila seven in absentia (Siah) inhibits cell growth: Suppression by BAG-1" EMBO JOURNAL, OXFORD UNIVERSITY PRESS, SURREY, GB, vol. 17, no. 10, 15 mai 1998 (1998-05-15), pages 2736-2747, XP002153981 ISSN: 0261-4189

## Description

La présente invention a pour objet des méthodes de détection, identification et/ou criblage de composés pouvant notamment être utilisés pour le traitement des cancers ou de certaines maladies neuro-dégénératives, liés à des dérèglements dans la régulation de la suppression tumorale et/ou de l'apoptose, dans la chaîne biologique de p53.

L'apoptose, ou mort cellulaire, est un phénomène complexe qui est régulé par de nombreuses protéines, dont la protéine p53. cette protéine interagit avec de nombreuses autres protéines, et son expression, qui induit les phénomènes de mort cellulaire et de réversion tumorale, peut être corrélée avec l'induction ou la répression de l'expression d'autres gènes cellulaires.

Les inventeurs de la présente invention ont ainsi mis en évidence des gènes induits et activés lors de la cascade menant à la répression tumorale et/ou l'apoptose (TSAP pour « Tumor Suppressor Activated Pathway »), ou des gènes réprimés (TSIP pour « Tumor Suppressor Inhibited Pathway »). Ces gènes ont fait notamment l'objet des demandes de brevets WO 97/22695 ou WO 00/08147.

Il est important de pouvoir comprendre précisément les mécanismes de la cascade de p53, afin de pouvoir générer de nouveaux composés ayant une activité antitumorale (pouvant notamment induire l'apoptose ou la suppression tumorale), ou pouvant être utilisés pour le traitement de maladies neuro-dégénératives. En effet, les inventeurs de la présente demande ont démontré que la préséniline 1 (PS1), dont la rôle avait été suggéré dans la maladie d'Alzheimer, était identique à la protéine TSIP 2 décrite dans la demande WO 97/22695. Ainsi, il est légitime de rechercher des médicaments pouvant interférer dans l'apoptose, afin de réduire ce phénomène, et qui pourraient être utilisés dans les maladies neuro-dégénératives.

La présente invention se rapporte à des procédés de criblage et d'identification de produits pouvant interférer dans la cascade de p53, et ainsi induire la réversion tumorale et/ou l'apoptose ou inversement, diminuer les phénomènes d'apoptose.

La présente invention est basée sur le fait que la protéine TSAP 3, décrite dans la demande de brevet WO 97/22695 se lie à la protéine Numb (telle que décrite dans la base de donnée GenBank, sous le numéro d'accession AF015040), qui présente elle-même des interactions avec Mdm2, une protéine impliquée dans les processus de contrôle de p53.

La présente invention est également basée sur le fait que la liaison de TSAP 3 (aussi identifiée comme étant la protéine Siah-1, appelée ainsi ou, de façon indifférente, Siah) à Numb induit la dégradation de la protéine Numb, par une voie dépendante du protéasome de l'ubiquitine.

Ainsi dans un premier mode de mise en oeuvre, la présente invention se rapporte à des procédés de criblage et/ou de sélection ou d'identification d'un composé interférant avec, réduisant ou inhibant la liaison de Siah à Numb, présentant les étapes de :
a) mise en contact dudit composé avec une cellule mammifère exprimant les protéines Siah et Numb ;
b) identification de l'augmentation de la quantité de protéine Numb dans ladite cellule mammifère par rapport à une cellule témoin avec laquelle ledit composé n'a pas été mis en contact.

Dans un second mode de mise en oeuvre, l'invention est dirigée vers un procédé de criblage, de sélection ou d'identification d'un composé interférant avec, réduisant ou inhibant la liaison de Siah à Numb, présentant les étapes de :
a) mise en contact dudit composé avec un système permettant la détermination *in vitro* de la liaison entre Siah et Numb ;
b) identification de la diminution et/ou l'inhibition de la liaison entre Siah et Numb.

Dans un autre mode de mise en oeuvre, les enseignements de la présente invention permettront d'identifier des composés qui, se liant à Numb, éventuellement à la place de Siah, vont induire une dégradation de Numb plus importante que la dégradation induite par la protéine Siah. En conséquence, la diminution de quantité de Numb impliquera une augmentation de la quantité de p53 présente, et une augmentation de la répression tumorale et/ou la mort cellulaire (apoptose). En conséquence, l'invention est dirigée vers un procédé de criblage, de sélection et/ou d'identification d'un composé pour l'augmentation de la répression tumorale et/ou la mort cellulaire (apoptose), présentant les étapes de :
a) mise en contact dudit composé avec une cellule mammifère exprimant les protéines Siah et Numb ;
b) identification de la diminution de la quantité de protéine Numb dans ladite cellule mammifère par rapport à une cellule témoin avec laquelle ledit composé n'a pas été mis en contact.

De façon inverse, la présente invention a notamment également pour objet un procédé de criblage, de sélection ou d'identification d'un composé pour la diminution et/ou l'inhibition de la répression tumorale et/ou la mort cellulaire (apoptose), présentant les étapes de :
a) mise en contact de composés avec un système permettant la détermination *in vitro* de la liaison entre Siah et Numb ;
b) identification des composés induisant la diminution et/ou l'inhibition de la liaison entre Siah et Numb ;
c) mise en contact des composés identifiés dans l'étape b) avec une cellule mammifère exprimant les protéines Siah et Numb ;
d) identification de l'augmentation de la quantité de protéine Numb dans ladite cellule mammifère par rapport à une cellule témoin avec laquelle ledit composé n'a pas été mis en contact.

Afin de déterminer des composés permettant l'augmentation de la répression tumorale et/ou la mort cellulaire (apoptose), il est également possible de combiner différents procédés selon l'invention, il est notamment possible d'obtenir ainsi un procédé de criblage, de sélection ou d'identification de composés ayant pour fonction une augmentation de la répression tumorale et/ou la mort cellulaire (apoptose), présentant les étapes de :
a) mise en contact dudit composé avec un système permettant la détermination *in vitro* de la liaison entre Siah et Numb ;
b) identification des composés induisant la diminution et/ou l'inhibition de la liaison entre Siah et Numb.
c) mise en contact des composés sélectionnés dans l'étape b) avec une cellule mammifère exprimant les protéines Siah et Numb ;
d) identification de la diminution de la quantité de protéine Numb dans ladite cellule mammifère par rapport à une cellule témoin avec laquelle ledit composé n'a pas été mis en contact.

Un tel procédé est donc également un objet de la présente invention.

La présente invention utilise donc le fait que les protéines Siah et Numb peuvent se lier l'une à l'autre. Il est donc intéressant d'identifier les domaines de chaque protéine qui sont effectivement en contact avec l'autre protéine. En effet, ceci devrait permettre de pouvoir utiliser les peptides ainsi identifiés en tant que leurres ou agonistes des protéines complètes. Ceci peut ainsi permettre de définir des composés qui interféreront dans la liaison Siah - Numb, et qui pourront soit induire la suppression tumorale et/ou l'apoptose (notamment si ces peptides ont un effet sur la dégradation de Numb(peptides issus de Siah)), soit à l'inverse diminuer ces phénomènes (notamment si ces peptides empêchent la liaison de Siah à Numb (soit qu'ils soient peptides leurres issus de Numb, ou qu'ils soient issus de Siah empêchent la liaison de cette protéine à Numb, notamment par compétition).

La présente invention a donc notamment pour objet un procédé d'identification d'une région de Siah active pour l'augmentation de la suppression tumorale et/ou de l'apoptose, comportant les étapes de :
a) mise en contact de peptides issus de la protéine Siah avec une cellule mammifère exprimant la protéine Numb ;
b) identification de la diminution de la quantité de protéine Numb dans ladite cellule mammifère par comparaison avec la quantité de protéine Numb présente dans une cellule mammifère témoin exprimant les protéines Siah et Numb.

Par « région de Siah », on entend notamment des peptides de séquence primaire issue de la séquence primaire de la protéine Siah.

La présente invention est donc également dirigée vers un procédé d'identification d'une région de Siah active pour la diminution et/ou l'inhibition de la suppression tumorale et/ou de l'apoptose, comportant les étapes de :
a) mise en contact de peptides issus de la protéine Siah avec une cellule mammifère exprimant la protéine Numb ;
b) identification de l'augmentation de la quantité de protéine Numb dans ladite cellule mammifère par comparaison avec la quantité de protéine Numb présente dans une cellule mammifère témoin exprimant les protéines Siah et Numb.

De façon plus générale, la présente invention permet l'identification de régions de Siah impliquées dans la liaison avec la protéine Numb, par un procédé comportant les étapes de :
a) mise en contact de peptides issus de la protéine Siah dans un système permettant la détermination *in vitro* de la liaison entre Siah et Numb ;
b) identification des peptides entraînant la diminution de la liaison entre Siah et Numb dans ledit système ;

Il est évident que la présente invention permet également la détermination des régions de Numb impliquées dans la liaison avec Siah, selon des procédés semblables aux procédés décrits précédemment que ces régions peuvent notamment être utilisées comme leurres lorsque l'on désire diminuer la répression tumorale et/ou l'apoptose.

La présente invention permet donc d'identifier des produits permettant d'interagir avec la liaison Siah - Numb, et donc pouvant avoir un intérêt dans la régulation de l'apoptose et/ou la répression tumorale. Toutefois, il est possible que ces produits, pour pouvoir être utilisés pour un traitement thérapeutique, notamment le cancer ou les maladies neuro-dégénératives, nécessitent d'être optimisés, afin d'avoir une activité supérieure, et/ou une toxicité moindre.

En effet, le développement d'un médicament est souvent effectué sur le principe suivant :
- criblage de composés ayant une activité voulue par une méthode approprié,
- sélection des composés répondant au « cahier des charges » (ici, une action sur les phénomènes d'apoptose et de réversion tumorale),
- détermination de la structure (notamment la séquence (éventuellement tertiaire) s'il s'agit de peptides, formule et squelette s'il s'agit de composés chimiques) des composés sélectionnés,
- optimisation des composés sélectionnés, par modification de la structure (par exemple par le changement de la conformation stéroéochimique (par exemple passage de L en D des acides aminés dans un peptide), ajout de substituants sur les squelettes peptidiques ou chimiques, notamment par greffage de résidus sur le squelette, modification des peptides (voir notamment Gante ("Peptidomimetics", dans Angewandte Chemie-Intemational Edition Engl, 1994, 33. 1699-1720),
- passage et criblage des composés ainsi obtenus sur des modèles appropriés qui sont souvent des modèles plus proches de la pathologie étudiée. A ce stade, on utilise notamment souvent des modèles animaux, en général chez les rongeurs (souris, rats...) ou chez des chiens, voire des primates.

Les modèles animaux qui peuvent être utilisés sont par exemple, pour le cancer, des modèles basés sur des souris immunodéprimées (par exemple *scid*/*scid),* auxquelles on injecte (notamment en sous-cutané) des cellules tumorales, qui vont mener au développement de tumeurs. On étudie l'efficacité des composés potentiellement anti-tumoraux, par exemple par la mesure de la taille des tumeurs formées.

On peut, pour l'étude des maladies neuro-dégénératives, utiliser le modèle décrit par Amson *et al.* (2000, Proc. Natl. Acad. Sci. USA, 97, 5346-50), qui consiste en des souris déficientes en p53, ou le modèle décrit dans *Chen et al.,* Janus *et al.,* et *Morgan et al.* (2000, Nature, 408 pp. 975-985)

Ainsi, la présente invention a notamment pour objet de permettre l'identification de composés qui pourraient être utilisés pour le traitement du cancer, en ce qu'ils possèdent une activité d'augmentation de la répression tumorale et/ou de l'apoptose. Un des objets de la présente invention est donc un procédé comportant les étapes de :
a) mise en oeuvre d'un procédé selon l'invention, permettant d'identifier des composés ayant une certaine activité d'augmentation de la répression tumorale et/ou de l'apoptose,
b) modification du produit sélectionné à l'étape a),
c) test du produit modifié dans l'étape b) dans des procédés *in vitro* et/ou *in vivo,* sur des modèles pertinents de répression tumorale et/ou d'apoptose,
d) identification du produit permettant d'obtenir une activité de répression tumorale et/ou d'apoptose supérieure à l'activité obtenue pour le produit sélectionné à l'étape a).

L'étape d) peut être remplacée par une étape d'), qui serait :
d') identification du produit permettant d'obtenir l'effet biologique cherché avec une toxicité moindre dans un modèle animal (lorsque un des modèles utilisés dans l'étape c) est *in vivo).*

Lorsque l'on effectue les tests sur des modèles d'apoptose ou de répression tumorale *in vitro,* on peut par exemple utiliser le modèle K256/KS décrit par Tellerman *et al.* (1993, Proc. Natl. Acad. Sci. USA, 90, 8702-6). On peut également utiliser les cellules Ml- LTR, décrites par Amson *et al.* (1996, Proc. Natl. Acad. SCI ; USA, 93, 3953-7), ou les cellules U937/US3-US4, décrites par Nemani *et al.* (1996, Proc. Natl. Acad. Sci. USA, 93, 9039-42).

Les essais *in vivo* peuvent être réalisés en injectant ces cellules des animaux, notamment des souris immunodéprimées et en étudiant les effets des différents composés testés.

L'homme du métier saura définir les conditions et les seuils nécessaires pour l'identification d'un produit pouvant être utilisé en tant que médicament, selon les exigences réglementaires (notamment de toxicologie), par rapport au bénéfice apporté par le produit ainsi identifié.

De la même façon, l'invention concerne également les procédés d'optimisation des produits réprimant la répression tumorale et/ou l'apoptose, identifiés par les procédés décrits plus haut, et permettant l'identification de produits utilisables comme médicaments.

Ainsi l'invention concerne également un procédé d'identification d'un produit ayant une activité de diminution et/ou d'inhibition de la répression tumorale et/ou de l'apoptose, caractérisé en ce qu'il comporte les étapes de :
a) mise en oeuvre d'un procédé selon l'invention, permettant d'identifier des composés ayant une certaine activité de diminution de la répression tumorale et/ou de l'apoptose,
b) modification du produit sélectionné à l'étape a) notamment par greffage de résidus sur le squelette chimique,
c) test du produit modifié dans l'étape b) dans des procédés *in vitro* et/ou *in vivo,* sur des modèles pertinents de répression tumorale et/ou d'apoptose,
d) identification du produit permettant d'obtenir une activité de répression tumorale et/ou d'apoptose réduite par rapport à l'activité obtenue pour le produit sélectionné à l'étape a).

L'étape d) peut également être remplacée par une étape d'), qui serait :
d') identification du produit permettant d'obtenir l'effet biologique cherché avec une toxicité moindre dans un modèle animal (lorsque un des modèles utilisés dans l'étape c) est *in vivo).*

Il s'agit en effet encore de pouvoir obtenir le produit qui présente le meilleur rapport (activité biologique et effet clinique) / (risques potentiels d'utilisation).

Les paramètres à mettre en jeu pour obtenir ces résultats sont tous connus et à la portée de l'homme du métier, désirant développer de nouveaux médicaments, et peuvent être trouvés par exemple dans les directives des organismes tels que l'Agence du Médicament, la Commission Européenne, ou la Federal Drug Agency.

La mise en oeuvre des procédés selon la présente invention nécessite des modèles permettant de déterminer la liaison entre Siah et Numb, ainsi qu'une mesure aisée des augmentations ou diminution de la quantité de protéine Numb dans une cellule eucaryote.

La quantité de protéine Numb peut être étudiée par Western Blot, la révélation s'effectuant par utilisation d'un anticorps dirigé contre ladite protéine. De tels anticorps peuvent notamment être obtenus au Weizmann Institute of Science, Rhovot, Israël, sous la référence WIS 3465, ou chez Transduction Laboratories (référence N80220).

Afin de mettre en oeuvre les procédés selon l'invention nécessitant l'étude et le criblage sur des cellules mammifères, il peut être avantageux de surexprimer l'une ou l'autre des protéines Siah et Numb dans lesdites cellules, ou les deux ensemble.

Ainsi, il est plus facile de pouvoir étudier les variations dans la quantité de protéine Numb, notamment par comparaison des cellules sur lesquelles on teste les composés d'intérêt avec les mêmes cellules, auxquelles on n'ajoute pas les composés que l'on désire cribler, et des cellules qui expriment Numb, sans exprimer Siah (contrôles positifs).

Il est entendu que l'expression des deux protéines Siah et Numb peut être augmentée par introduction des gènes (notamment les ADNc) codant pour ces deux protéines dans les cellules, soit placés sur des vecteurs, soit par introduction dans le chromosome.

Lorsque l'on choisit une expression épisomale, ladite cellule mammifère est transfectée par au moins un vecteur choisi parmi un vecteur portant un fragment d'ADN codant pour Siah, un vecteur portant un fragment d'ADN codant pour Numb, un vecteur portant un fragment d'ADN pour Siah et un fragment d'ADN codant pour Numb. Ainsi, le même vecteur peut exprimer les deux protéines, alternativement il est possible d'introduire deux vecteurs.

Il est possible d'utiliser des vecteur permettant l'expression et la purification facile des protéines Siah et Numb, par exemple dans des cellules procaryotes (E. *coli, B. subtiis* ...) ou eucaryotes (levures telles *Saccharomyces, Kluyveromyces...,* cellules mammifères (HeLa, Cos, Hep-2 ... ) ou d'insectes (en utilisant un système à Baculovirus). Ainsi, il peut être avantageux que les protéines possèdent une étiquette à leur extrémité N- ou C-terminale, afin de faciliter la purification. On choisit notamment une étiquette Histidine, ou GST. Ces méthodes sont bien connues de l'homme du métier, qui trouve les plasmides appropriés dans les catalogues de sociétés telles Stratagène.

Lorsque l'on met en oeuvre les procédés selon l'invention sur des modèles *in vitro,* pour étudier la liaison entre Numb et Siah, il existe plusieurs façons d'opérer.

Un protocole utilisable peut être le suivant :
- expression et purification des protéines Siah et Numb, par exemple dans des cellules procaryotes *(E. coli, B. subtiis...)* ou eucaryotes (levures telles *Saccharomyces, Kluyveromyces...,* cellules mammifères (HeLa, Cos, Hep-2...) ou d'insectes (en utilisant un système à Baculovirus). Il peut être avantageux que les protéines possèdent une étiquette à leur extrémité N- ou C-terminale, afin de faciliter la purification. On choisit notamment une étiquette Histidine, ou GST. Ces méthodes sont bien connues de l'homme du métier, qui trouve les plasmides appropriés dans les catalogues de sociétés telles Stratagène.
- liaison des protéines à des billes appropriées. Lorsque l'on utilise une étiquette GST, on lie les protéines exprimées à des billes de sépharose présentant de la Gluthathione.
- préparation de protéines par traduction *in vitro.* Ceci peut aisément être effectué en utilisant des vecteurs commerciaux (par exemple disponibles chez Promega), qui permettent de cloner les ADNc sous le contrôle de promoteurs bien connus (T7 ou T3), et d'utiliser les ARN polymérases appropriées pour produire les ARN, puis d'effectuer l'expression des protéines *in vitro.,* en utilisant les trousses disponibles, et en suivant les indications du fabriquant.
- co-précipitation des protéines, en ajoutant les protéines obtenues par traduction *in vitro* aux billes de sépharose - glutathione sur lesquelles sont fixées les protéines de fusion avec la GST. Après un temps de contact suffisant, on lave les billes et on effectue une analyse par gel d'électrophorèse en SDS-PAGE, et autoradiographie. L'apparition des bandes correspondant aux deux protéines montre bien la liaison entre celles-ci.

L'utilisation de contrôles appropriés permet ainsi de définir la diminution et/ou l'inhibition de la liaison entre Siah et Numb, par comparaison des quantités de protéines libérées après ajout du composé testé lors de l'étape de coprécipitation avec les quantités de protéines libérées dans les contrôles.

On peut aussi étudier la liaison des protéines en utilisant le système FRET (Fluorescence Resonance Energy Transfer) qui consiste à marquer chacune des protéines avec un résidu approprié, la liaison des deux protéines induisant une réaction entre chacun des deux résidus et l'émission d'une fluorescence aisément détectable.

Il est clair que le terme séquence peptidique ou séquence nucléique ou nucléotidique (ces deux derniers termes étant utilisés de façon indifférente) représentent des séquences isolés, c'est-à-dire hors de leur état naturel, et peuvent notamment être modifiés par le remplacement de leurs unités de bases par des unités non-naturelles, ou par la modification des liaisons entre les unités de bases (par exemple les phosphorothioates (acide nucléique) ou les Peptid Nucleic Acids)

Il est un objet de l'invention que de permettre l'identification de composés interférant avec la liaison Siah et Numb, certains de ces composés pouvant notamment induire des effets sur la cascade de p53. Ainsi, les composés selon l'invention, les séquences peptidiques selon l'invention, ou les séquences nucléotidiques selon l'invention, à titre de médicament, sont également objets de l'invention.

Un composé identifié par une méthode selon l'invention peut être un composé ayant une structure chimique, un lipide, un sucre, une protéine, un peptide, un composé hybride protéine-lipide, protéine-sucre, peptide-lipide, ou peptide-sucre, une protéine ou un peptide sur lequel on a ajouté des ramifications chimiques.

Parmi les composés chimiques envisagés, ils peuvent contenir un ou plusieurs cycles, aromatique(s) ou non, ainsi que plusieurs résidus de toute sorte (notamment alkyle inférieur, c'est-à-dire présentant entre 1 à 6 atomes de carbones).

Ces composés, séquences nucléiques, séquences peptidiques, peuvant ainsi être utilisés pour la préparation d'un médicament, destiné notamment au traitement du cancer, ou d'une maladie neuro-dégénérative, selon l'effet pro- ou anti-apoptose/réversion tumorale.

Les inventeurs de la présente demande ont, pour la première fois, mis en évidence le fait que la protéine Siah se lie à la protéine Numb. Ainsi, la présente invention concerne également un complexe constitué d'une protéine Siah et d'une protéine Numb.

La présente invention concerne également un procédé d'inhibition de la liaison de Siah à Numb dans une cellule, comprenant l'étape de :
a) mise en contact de ladite cellule avec un composé identifié par un procédé selon l'invention, inhibant la liaison Siah-Numb.

Le composé ainsi envisagé peut également être un peptide « leurre » issu de la protéine Siah ou de la protéine Numb. Le procédé peut être mis en oeuvre *in vitro* ou *in vivo.*

La présente invention est également dirigé vers une méthode pour le traitement d'un cancer, caractérisée en ce que l'on administre, à un patient, un composé identifié selon la présente invention et augmentant l'apoptose et/ou la réversion tumorale.

Une méthode pour le traitement d'une maladie neuro-dégénérative, consistant à administrer, à un patient, un composé selon la présente invention et diminuant ou inhibant l'apoptose, est également un objet de la présente invention.

Ainsi que précisé et démontré dans les exemples, la protéine Siah, non seulement se lie à la protéine Numb, mais induit également sa dégradation selon la voie du protéasome lié à l'ubiquitine. Il est remarquable de noter que la protéine Numb est connue pour se lier à la protéine Mdm2, dont la présence induit une régulation négative de p53. Il est par ailleurs connu que l'expression de Siah dans des cellules induit des phénomènes d'apoptose, ainsi que l'ont précédemment démontré les inventeurs. Ainsi, la présente invention fournit aussi un procédé d'inhibition de la diminution de p53, notamment liée à Mdm2 *via* Numb dans une cellule, comprenant l'étape de :
a) mise en contact de ladite cellule avec un composé selon l'invention, qui augmente l'apoptose et/ou la réversion tumorale.

De façon inverse, on peut augmenter la diminution de quantité de protéine p53 dans une cellule, notamment en raison des interactions entre Mdm2 et Numb, comprenant l'étape de :
a) mise en contact de ladite cellule avec un composé selon l'invention, qui inhibe ou diminue l'apoptose et/ou la répresson tumorale.

Ces deux procédés peuvent être mis en oeuvre *in vitro* ou *in vivo,* notamment dans le cadre d'un traitement thérapeutique.

### LEGENDES DES FIGURES

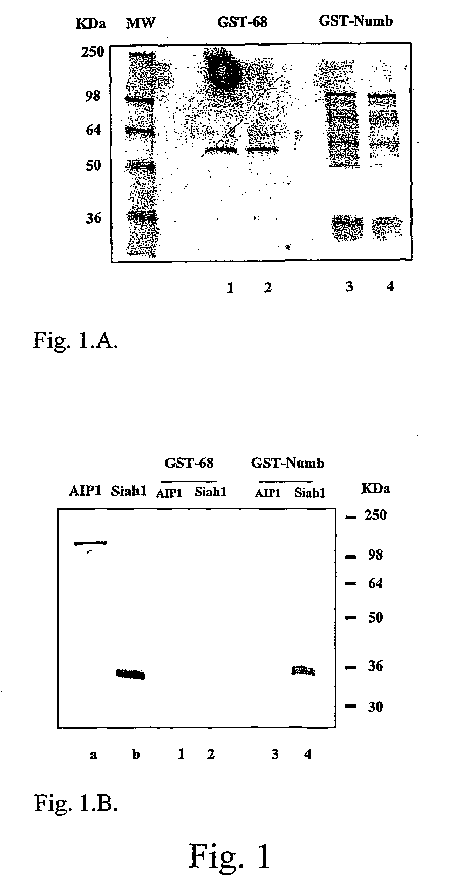

**Figure 1** : analyse de l'interaction *in vitro* entre Siah-1 et Numb. Figure 1.A Protéines de fusion GST-68 (la protéine 68 étant le fragment Leu 82-Glu 314 de BNIP 2) (colonnes 1 et 2) et GST-Numb (colonnes 3 et 5), observées avec Bleu de Coomassie. Figure 1.B. Autoradiographe des produits de traduction *in vitro* des protéines AIP1 (colonne a), et Siah-1 (colonne b), marquées avec des méthionines ³⁵S. Les colonnes 1 à 4 illustrent la précipitation de AIP1 (1 et 3) ou Siah-1 (2 et 4) avec les produits indiqués par les protéines de fusion GST.

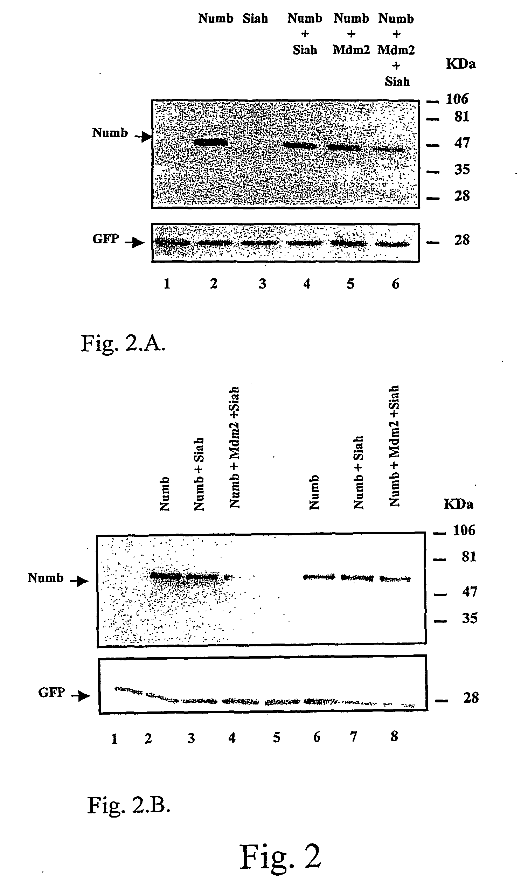

**Figure 2 :** analyse de l'effet de l'interaction *in vivo* entre Siah et Numb, sur les niveaux d'expression des protéines. Figure 2.A. Des cellules 293T ont été cotransféctées par un vecteur d'expression vide (colonne 1), ou exprimant Numb (colonne 2), Siah-1 (colonne 3), Numb et Siah-1 (colonne 4), Numb et Mdm2 (colonne 5), Numb, Siah-1 et Mdm2 (colonne6). 48 heures après transfection, une analyse a été effectuée par Western Blot, en utilisant un anticorps anti-Numb. Un contrôle a également été effectué en utilisant un anticorps anti-GFP (Green Fluorescent Protein) en tant que standard de charge sur le gel. Figure 2.B. l'analyse Western Blot a été effectuée comme décrit précédemment, en absence ou présence d'un inhibiteur du protéasome de l'ubiquitine (MG 132). Sans MG 132, la quantité de protéine Numb décroît en présence de Siah-1, avec ou sans Mdm2 (lignes 2, 3, 4). Avec MG 132, la quantité de protéine Numb reste constante (lignes 6, 7 et 8).

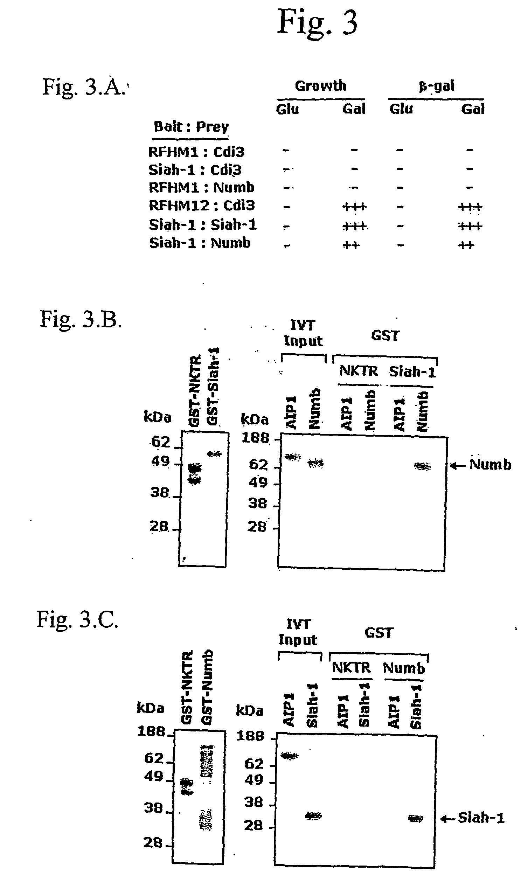

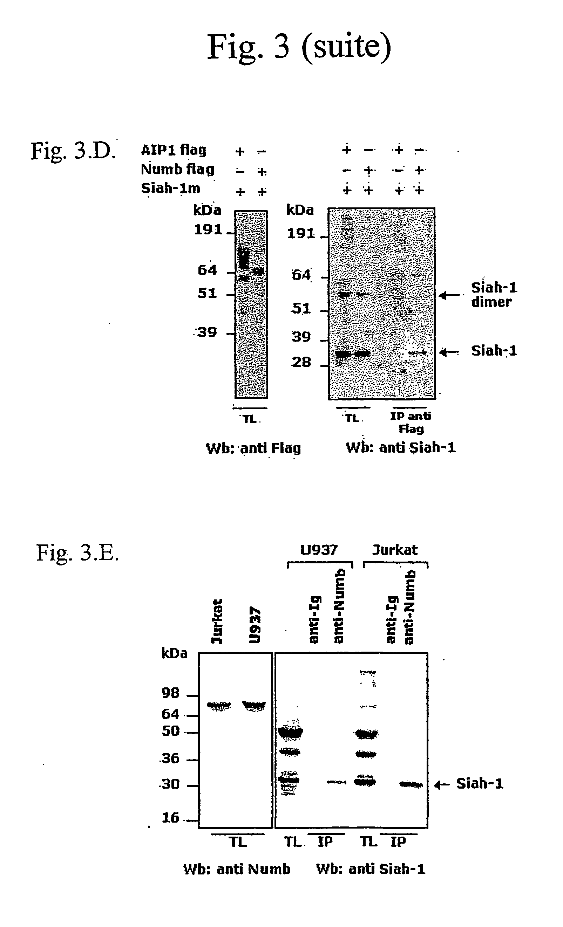

**Figure 3 :** analyse de l'interaction *in vivo* et *in vitro* de Siah-1 et Numb
   **A.** Résumé des données de levure en double hybride. Siah-1 et Numb interagissent par un essai d'interactions dans une levure en double hybride. Les interactions LexA-Siah-1 et B42-Numb ont été testées soit par croissance, soit par l'activité β-galactosidase. En présence de galactose (Gal) les cibles contenant B42 étiquetées pour l'activation sont exprimées tandis que sur du glucose (Glu) elles sont réprimées. Des levures contenant des combinaisons de plasmides cibles et de plasmides leurres en tant que contrôle négatif LexA-RFHM1/B42-Cdi3, Lex A-Siah-1/B42-Cdi3, LexA-RFHM1/B42-Numb ou des combinaisons de contrôle positif LexA-RFHM12/Cdi3 et LexA-Siah-1/B42-Siah-1 ont été testées en parallèle. Comme attendu, les levures contenant les plasmides cibles et leurres en tant que contrôle positif, ont présenté une croissance et étaient β-gal⁺ en présence galactose mais pas de glucose. De plus, les levures contenant LexA-Siah-1 et B42-Numb montraient également une forte croissance dépendante du galactose et une activité β-gal.
   **B.** Interaction *in vitro* de GST-Siah-1 et Numb radiomarqué. Numb et le contrôle négatif AIP1 ont été générés par traduction *in vitro* (IVT) dans un lysat de réticulocyte de lapin en présence de méthionine et cystéine marquées au ³⁵S. Des quantités égales de produits radiomarqués ont été incubées avec soit GST-Siah-1, soit des protéines de fusion GST-NKTR en tant que contrôle négatif capturées sur des billes de glutathion. Les protéines radiomarquées ont été éluées dans un tampon d'échantillon protéique, résolu dans des conditions de réduction (0,7 mM de 2-β-mercaptoéthanol), par électrophorèse sur gel de sodium dodécyl sulfate-polyacrylamide (SDS-PAGE) (10 %) et visualisé par autoradiographie.
   **C.** Association réciproque entre GST-Numb et Siah-1. AIP1 ou Siah-1 dérivé d'une traduction *in vitro* (IVT) ont été générés et des essais d'abaissement de GST ont été réalisés avec soit GST-NKTR, soit GST-Numb comme décrit ci-dessus. La représentation de la contribution du GST indiqué est décrite sur les panneaux de gauche de B et C. A noter que GST-Numb est partiellement dégradé.
   **D.** Interaction *in vivo* de Flag-Numb et Siah-1. Une construction Siah-1m portant 3 mutations faux sens (Cys → Ser) au niveau des positions en acide aminé 129, 131 et 135 dans Siah-1 a été générée de façon à donner lieu à une protéine plus stable. Des cellules 293T ont été cotransfectées de façon transitoire avec soit Flag-AIP1 et Siah-1m soit Flag-Numb et Siah-1m par la méthode de la précipitation au phosphate de calcium. Les niveaux d'expression de Flag-AIP1 et Flag-Numb dans les lysats cellulaires totaux (TL) ont été déterminés par immunomarquage avec un anticorps anti-flag (D, panneau de gauche). Les extensions d'association Siah-1-Numb ont été déterminées par immunoprécipitation de Flag-Numb avec un anticorps Flag, suivi par immunomarquage avec un anti-sérum contre Siah-1 (D, panneau de droite). A noter que la forme monomère de Siah-1 migre comme un peptide d'environ 32 kDa. La bande protéique d'environ 60 kDa dans le lysat total de Siah-1 représente probablement des dimères de Siah-1.
   **E.** Interaction endogène de Siah-1 et Numb dans des cellules U937 et des cellules Jurkat. 2,5 X 10⁸ cellules ont été chimiquement liées par croisement avec le lieur DTBP réductible. Les lysats dérivés de cellules ont été incubés toute la nuit avec soit un anti-Ig qui apparie les espèces en tant que contrôle négatif, soit un anticorps anti-Numb, suivi par une incubation avec des protéines -G-agarose. Les complexes immuns ont été résolus dans un gel SDS-PAGE à 12 % et ensuite marqués avec un anticorps anti-Siah-1. A noter que l'anticorps anti-Numb co-immunoprécipite la forme monomère 32 kDa de Siah-1 à la fois dans les cellules U937 et dans les cellules Jurkat (E, panneau de droite). Le panneau de droite montre l'expression endogène de Numb dans les 2 lysats cellulaires (TL).

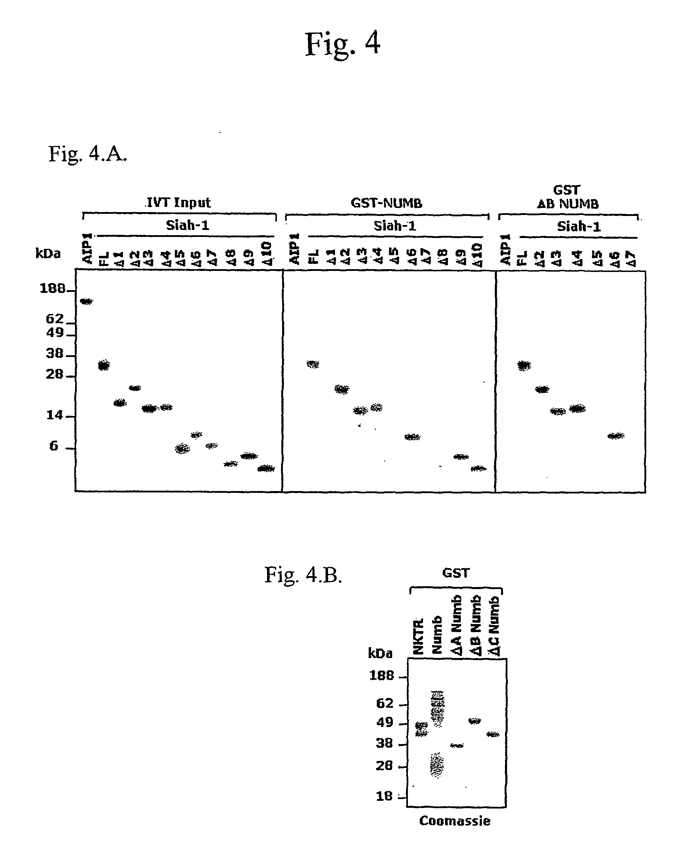

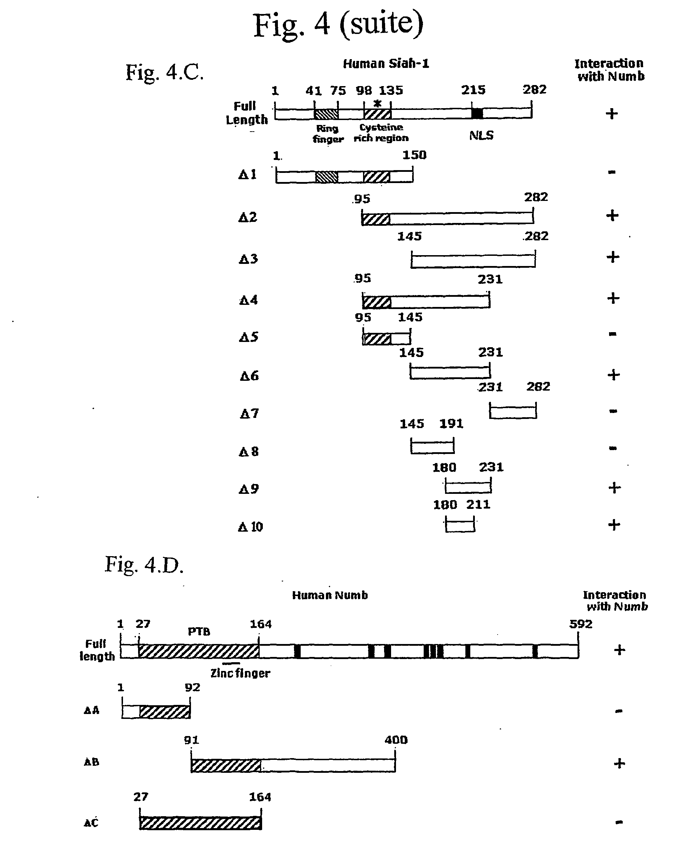

**Figure 4 :** Cartographie des domaines d'interaction Siah-1 et Numb
   **A.-C.** Des essais d'abaissement de GST ont été réalisés par incubation des fragments indiqués de Siah-1 dérivés d'une traduction *in vitro* (IVT) radiomarqués (Δ1-10) ou du contrôle négatif AIP1 avec GST-NKTR (donnée non montrée) ou de plusieurs protéines de fusion GST de Numb (ΔA-C). Des échantillons ont été résolus tels que décrit ci-dessus. A noter que GST-Numb (second panneau) interagit avec un fragment minimal de Siah-1 Δ10 composé des acides aminés 180-211.
   **B.** Une représentation de la contribution de GST.
   **C. et D.** Diagramme représentant les mutants de délétion Siah-1 et Numb, respectivement. Les barres noires montrées chez Numb dans sa pleine longueur repèrent des motifs contenant SH3.
   (*) dans C. indique la localisation des 3 mutations faux sens.

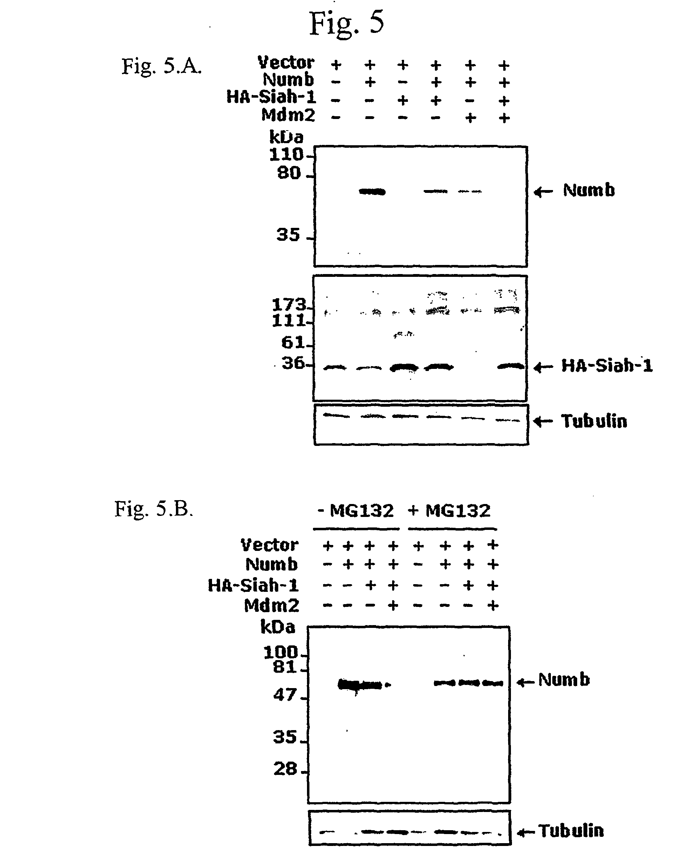

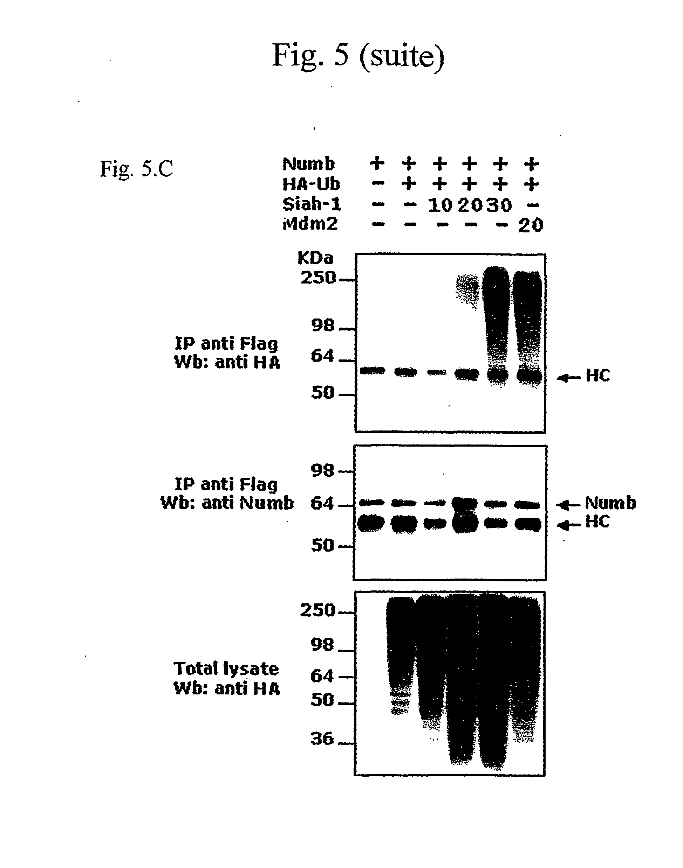

**Figure 5:** Effets de la surexpression de Siah-1 sur la dégradation de Numb et l'ubiquitination.
   **A.-C.** Siah-1 induit la dégradation de Numb *in vivo.*
   **A.** Des cellules 293T ont été cotransfectées de façon transitoire avec les plasmides indiqués, par la méthode de la précipitation au phosphate de calcium. 48 heures après la transfection, les lysats cellulaires totaux ont été préparés et résolus par une analyse SDS-PAGE suivie par des anticorps. Pour vérifier la charge équivalente des échantillons protéiques, les membranes ont été décapées et resondées avec un anticorps anti-tubuline (A., panneau inférieur).
   **B.** Les niveaux de l'état stable de Numb ont été analysés par analyse par immunomarquage soit en présence, soit en absence de l'inhibiteur de protéasome, MG132 (50 µM), ajouté 3 heures avant la récolte des cellules.
   **C.** *Ubiquitination in vivo* de Numb par Siah-1. Les cellules 293T ont été transfectées de façon transitoire avec des plasmides codant pour flag-Numb, HA-polyubuiquitine et des quantités variables de Siah-1 (10-30 µg). 24 heures après la transfection, les cellules ont été incubées avec un anticorps anti-flag. Les immunoprécipités ont été analysés pour l'ubiquitination par immunomarquage avec un anti-HA. A noter que le MG132 (50 µM) a été ajouté à des transfectants 293T 6 heures avant la récolte dans le but d'empêcher la dégradation de Numb. HC repère la chaîne lourde de l'immunoglobuline (C, panneau supérieur). Mdm2 a été utilisé comme point de référence de contrôle positif. Le panneau du milieu montre les niveaux d'expression de flag-Numb tel que révélé par l'anticorps anti-Numb. Le panneau inférieur montre le schéma global de l'ubiquitination tel que détecté par l'anti-HA avec des quantités croissantes de Siah-1 ou Mdm2.

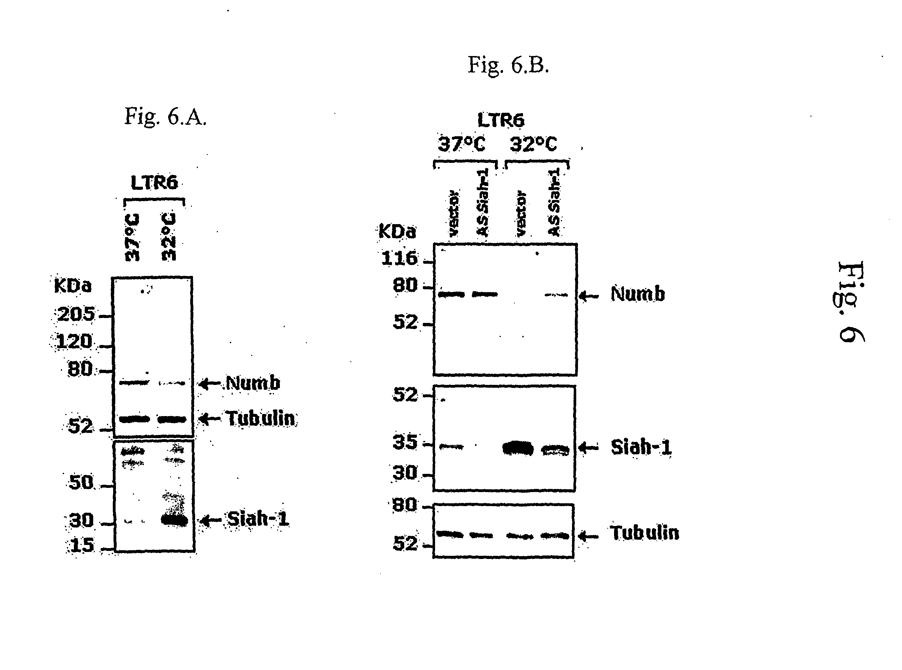

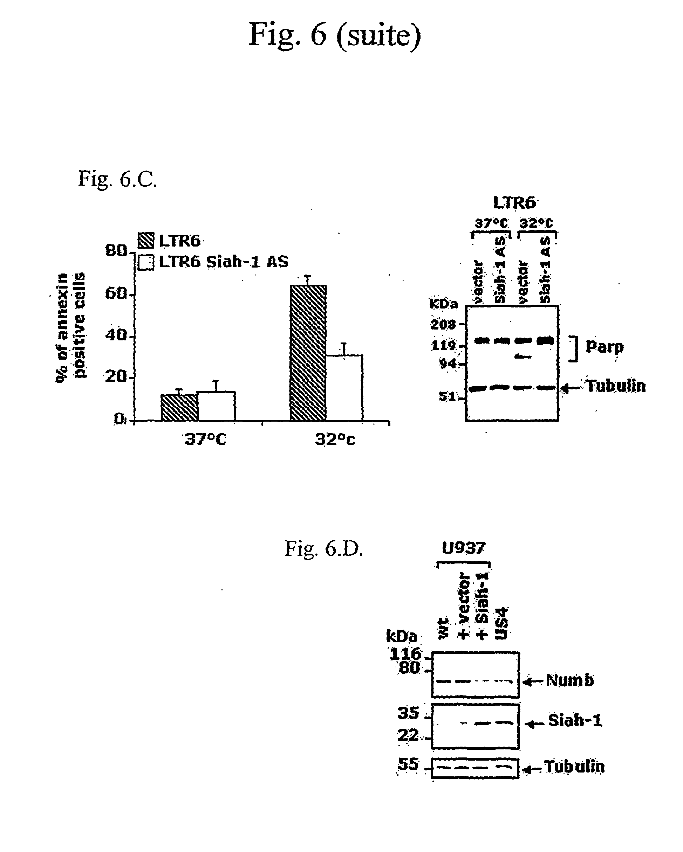

**Figure 6 :** Effets *in vivo* de Siah-1 sur la dégradation endogène de Numb
   **A.-D.** La potentialisation de Siah-1 induite par p53 favorise des niveaux diminués de Numb endogène.
   **A.** 24 heures après avoir passé les cellules LTR6 à 32°C, ce qui donne lieu à une activation de p53, les lysats cellulaires ont été préparés et analysés concernant les niveaux d'expression de Siah-1 endogène (A. panneau inférieur) et Numb endogène (panneau supérieur). A noter que les niveaux de protéines Siah-1 accrues sont concomitants avec une diminution de l'expression de Numb.
   **B.** Analyse des cellules LTR6 transfectées de façon stable avec un anti-sens Siah-1. Déjà à 37°C, l'anti-sens Siah-1 inhibait l'expression de la protéine Siah-1 de 32 kDa (B. panneau du milieu, bande 2). La croissance de Siah-1 suivant l'activation de p53 (B. panneau du milieu, bande 3) a été inhibée de façon significative dans les cellules transfectées avec une construction anti-sens. Ceci a réduit l'expression de Siah-1 qui résultait du niveau accru de la protéine Numb (B. panneau supérieur, bande 4).
   **C.** Le panneau de gauche montre une analyse FACS de cellules positives à l'annexine-v suivant l'activation de p53 dans les cellules LTR6 soit transfectées avec un vecteur seul, soit avec l'anti-sens Siah-1. A noter, la réduction significative de l'apoptose induite par p53 avec l'anti-sens Siah-1.
   **C.** Panneau de droite, les lysats totaux cellulaires LTR6 ont été préparés et résolus par une analyse SDS-PAGE suivie par un immunomarquage avec soit un anticorps anti-PARP, soit un anticorps anti-tubuline.
   **D.** Les cellules U937 ou des transfectants stables U937 exprimant soit le vecteur contrôle, soit Siah-1 ont été analysées pour Numb endogène dans les lysats cellulaires totaux. De plus, le clone US4 dérivé des cellules U937 a également été analysé pour les niveaux protéiques endogènes Siah-1 et Numb. Des lysats ont été sondés avec soit les anticorps anti-Numb (D. panneau du haut), soit les anticorps anti-Siah-1 (D. panneau central), soit les anticorps anti-tubuline (D. panneau inférieur). A noter la réduction des niveaux protéiques Numb dans Siah-1 surexprimant les cellules U937.

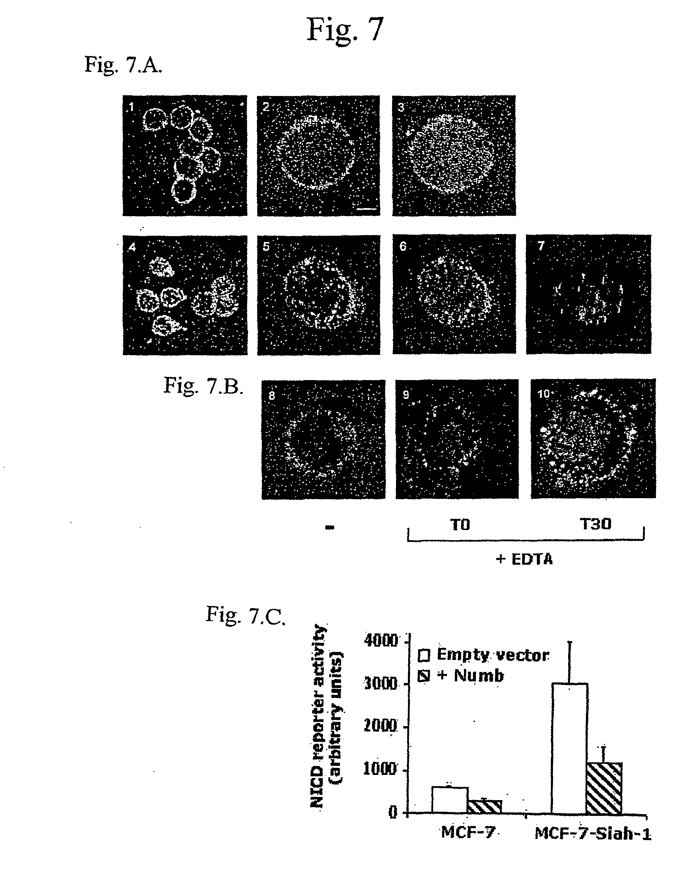

**Figure 7 :** Effets *in vivo* de la surexpression de Siah-1 sur l'activité Notch. Redistribution de Notch1 endogène dans les cellules U937 surexprimant Siah-1.
   **A.** Des transfectants stables U937 exprimant soit le vecteur contrôle (panneaux 1 à 3), soit Siah-1 (panneaux 4 à 7) ont été analysés par un microscope confocal pour l'immunofluorescence endogène de Notch1. Les cellules ont été marquées avec un anticorps reconnaissant la portion cytoplasmique de Notch1 humain, suivi par un anticorps secondaire conjugué à FITC. L'analyse d'immunofluorescence de Notch est rapportée soit à faible grossissement (panneaux 1 et 4), soit à fort grossissement (panneaux 2, 3, 5, 6 et 7). A noter le marquage type bordure du schéma de Notch1 dans les cellules U937 de contrôle vecteur comparé au marquage nucléaire et cytoplasmique dans les cellules surexprimant Siah-1. Le marquage à l'iodure de propidium a été inclus pour mieux visualiser le marquage nucléaire de Notch1 (panneaux 3, 6 et 7). La présence de Notch1 dans le noyau a été confirmée par l'image Z-plane (panneau 7). La barre de longueur représente 5 µm.
   **B.** Les panneaux 8 à 10 montrent la translocation nucléaire de Notch1 induite par EDTA dans les cellules U937 de contrôle vecteur (panneaux 8 à 10). Des cellules ont été incubées dans 10 mM de EDTA contenant du PBS pendant 30 min à 37°C, lavées et marquées avec des anticorps anti-Notch1 (intracellulaire), soit immédiatement (T=0), soit 30 min (T=30) après l'élimination de l'EDTA. Les cellules ont été contremarquées avec de l'iodure de propidium pour visualiser le noyau. A noter la présence de marquage positif de Notch1 dans le noyau à T=30. Le panneau 8 montre les cellules U937 non traitées.
   **C.** Activité Notch1. Des cellules MCF-7 et des transfectants stables MCF-7 qui surexpriment Siah-1 ont été transfectées de façon transitoire avec une construction reporter NICD (pGA981-6) et 48 heures après l'infection, elles ont été surveillées pour l'activité NICD. A noter l'activité reporter NICD endogène renforcée dans les cellules MCF-7 transfectées de façon stable avec Siah-1. Cette activité NICD accrue a été inhibée de façon efficace par une co-transfection avec Numb.

### EXEMPLES

### Exemple 1 : expression des protéines de fusion GST

Préparation d'une préculture à partir d'une colonie isolée de B121 (DE3), transformée avec le plasmide pGEX-P-1-Siah ou pGEX-P-1-Numb, dans un milieu SB avec 100µg/ml d'ampicilline, à 37°C.

Le plasmide PGEX-P-1 est disponible chez Amersham Pharmacia Biotech AB.

Les protéines sont les protéines humaines, codées par les ADNc complémentaires correspondant aux séquences SEQ ID N° 1 (Siah) et SEQ ID N° 2 (Numb). Les références dans GenBank des différentes protéines sont, pour Siah humain, HSU76247, et pour Numb humain, AF015040.

Le lendemain, inoculer 250 ml de SB+Amp avec 5 ml de la préculture.

Croissance à 37°C pour GST-Numb, ou 28°C pour GST-Siah, jusqu'à une densité optique entre 0,5 et 0,7.

Ajout de 0,1 mM IPTG pour induire la synthèse des protéines.

Croissance à à 37°C pour GST-Numb, ou 28°C pour GST-Siah pendant 1h30 (GST-Numb) ou 1h (GST-Siah).

Centrifugation 3000 rpm pendant 10 min (1800g, à 4°C).

Resuspension du précipité dans 10 ml de tampon A NP40 (NP40 1 % ; Tris pH 7,4 10 mM ; NaCl 150 mM ; EDTA 1mM; Glycerol 10% ; DTT 1mM ; Aprotinin 2µg/ml ; Leupeptin 2µg/ml ; Pepstatin 2µg/ml ; AEBSF 1mM).

Sonication 3 fois pendant 15 s à puissance 50, sur glace.

Centrifugation à 12000 rpm pendant 10 min (18000g à 4°C).

Garder le surnageant à -80°C.

### Exemple 2 - Liaison aux billes de sepharose-glutathione

Toute la préparation des billes et les incubations s'effectuent à 4°C.

Ajout de 2 ml de surnageant à 200 µl de billes (préparées après 3 rinçages dans le PBS, 1 rinçage dans le tampon A NP40, resuspension à 50 % (poids par volume) dans le tampon A NP40, centrifugation à 3000 rpm à chaque fois).

On utilise les billes Glutathione-sepharose 4B, fournies par Amersham Pharmacia Biotech AB, sous la référence 17.0756.01.

Remuer doucement pendant au moins 1 heure à 4°C.

Rincer les billes 3 fois dans le tampon A NP40 sans inhibiteur de protéase.

Resuspendre les billes dans 1 ml de tampon A NP40 avec inhibiteur de protéase.

Pour l'analyse par électrophorèse SDS-PAGE, prendre 20 à 40 µl des billes resuspendues, centrifuger pendant 5 min, écarter le surnageant, resuspendre les billes dans 10 µl de tampon de charge de X, chauffer à 97°C pendant 7 min. Charger sur gel et analyser après révélation par bleu de Coomassie, pour normaliser la quantité des protéines de fusion à utiliser.

### Exemple 3 : Traduction in vitro

Le kit TNT Coupled Reticulocyte Lysate System de Promega a été utilisé avec les ARN polymérase T7 ou T3 en fonction du vecteur utilisé pour traduire et exprimer les protéines (T7 pour AIPI et Siah 1, T3 pour Numb). Le kit a été utilisé selon les instructions du fabricant (référence L4610).

Les protéines ont incorporé de la méthionine S³⁵ (Amersham Pharmacia).

Les produits obtenus in vitro ont été analysés par électrophorèse SDS-PAGE.

Après électrophorèse, le gel est placé dans du tampon de fixation (5 % de méthanol, 15 % d'acide acétique, 80 % d'eau) pendant une 1/2 heure et le signal est amplifié par immersion du gel dans le produit Amplify de Amersham Pharmacia (référence NAMP 100).

Un film Kodak Biomax MR est alors exposé sur le gel séché pendant une durée variant d'une heure à une semaine, puis développé.

### Exemple 4 : Co-précipitation des protéines

30 µl des billes de sépharose- glutathione couplés aux protéines de fusion GST, après normalisation des quantités, ont été rincés dans le tampon B (NP40 1 %, TrisHCl 50 mM, NaCl150 mM, leupeptine 2 µl/ml, aprotinine 1 %, ABESF 1 mM). On ajoute ensuite 5 à 10 µl du produit de traduction *in vitro* tel qu'obtenu dans l'exemple 3, en fonction de la quantité du produit observé par autoradiographie.

Après une nuit de contact, les billes ont été rincées 10 fois avec du tampon A NP 40, sans antiprotéases.

On analyse par SDS-PAGE et autoradiographie.

La figure 1B montre donc clairement que la protéine Siah obtenue par traduction *in vitro* se lie à la protéine de fusion GST-Numb.

On peut montrer de la même façon que la protéine Numb obtenue par traduction *in vitro* se lie à la protéine de fusion de GST-Siah (non montré).

### Exemple 5 : Cellules et transfections transitoires

Les cellules humaines embryonnaires de rein 293T (Référence ATCC : CRL 1573) sont maintenues dans le DMEM supplémenté de 10 % de sérum de veau foetal (FCS) dans une atmosphère à 5 % en CO₂. Les cellules ont subi un nouveau passage le jour précédent la transfection.

Les transfections des cellules 293T ont été effectuées par la procédure du phosphate de calcium dans le DMEM plus 10 % FCS en présence de 20 µM de chloroquine. Huit heures après la transfection, le milieu de transfection a été remplacé par du DMEM plus 10 % FCS. Les cellules ont été recueillies 48 heures après la transfection.

Pour les tests avec le MG132, les cellules transfectées ont été traitées pendant 6 heures à 37°C avec du MG132 à 50 µM (Calbiochem-Novabiochem) dans du DMSO, ou avec du DMSO seul, comme contrôle. Les plasmides exprimant la protéine Numb humaine Mdm2 murine et la GFP étaient des plasmides pcDNA3 de chez Invitrogen, San Diego, CA, USA.

La protéine Siah-1 a été obtenue à partir de l'ADN complémentaire, sous-clonée dans le plasmide pBKRSV (Stratagene, La Jolla, CA, USA)

### Exemple 6 : Western Blot

48 heures après transfection des cellules 293T avec les plasmides, les cellules ont été lysées dans un tampon de lyse TBS-Triton (Tris-buffered saline [TBS], [pH 8,0] ; 1% Triton X-100, 10 mg/ml de phénylméthylsulfonyl fluoride ; aprotinine 5 mg/ml, leupeptine 5 mg/ml).

Les lysats cellulaires ont été séparés par gel d'électrophorèse SDS-PAGE et transférés sur une membrane de nitrocellulose (Biorad), suivis par un analyse par Western Blot avec l'anticorps polyclonal Numb (Institut Weizmann, réf. 3465, ou référence N80220 de la société Transduction Laboratories) et l'anticorps monoclonal anti-GFP (Boehringer, réf. 1814460), afin de vérifier la normalisation des quantités chargées sur le gel. Les signaux ont été détectés en utilisant un anticorps secondaire couplé à une peroxydase et à des réactifs électro chimioluminescents de chez Amersham Pharmacia Biotech AB.

La figure 2 montre bien que la quantité de protéine Numb diminue en présence de Siah, de la même façon qu'en présence de Mdm2. Ces effets semblent indépendants, car il existe une addition des effets lorsque les protéines Siah et Mdm2 sont toutes les deux mises en présence de Numb.

La figure 2B montre que la dégradation de Numb par la protéine Siah s'effectue par la voie du protéasome de l'ubiquitine, car elle est inhibée par l'utilisation d'un inhibiteur de cette voie.

Les résultats présentés dans la présente demande démontrent ainsi la liaison existant entre les protéines Numb et Siah, et le fait que la protéine Siah agit pour dégrader la protéine Numb, par une voie dépendante du protéasome de l'ubuiquitine.

On peut également montrer la liaison entre les protéines Siah et Numb, par utilisation d'autres techniques, notamment la technique du double-hybride, dérivé du système développé par Finley et Brent, (Annu Rev Genet 1997;31:663-704).

La protéine appât peut être clonée dans le plasmide pEG202 connu de l'homme du métier pour une telle application (promoteur 67-1511, lexA 1538-2227, ADH Ter 2209-2522, pBR remnants 2540-2889, 2µ, ori 2890-4785, YSCNFLP 4923-5729, HIS3 7190-5699, TYIB 7243-7707, RAF_part 7635-7976, squelette pBR 7995-10166, bla 8131-8988).

La protéine proie peut être clonée dans le plasmide pJG4-5, également bien connu de l'homme du métier (promoteur GAL 1-528, cassette de fusion 528-849, ADH Ter 867-1315, 2µ, ori 1371-3365, TRP1 3365-4250, squelette pUC 4264-6422, Ap 4412-5274).

On utilise également le plasmide rapporteur pSH18-34, également connu de l'homme du métier. Ce plasmide est notamment disponible chez Invitrogen, sous le numéro de référence V611-20, et également déjà transformé dans la souche EGY48 (aussi appelée RFY 231), chez le même fournisseur (référence souche seule : C835-00, transformée par pSH18-34 : C836-00).

La liaison est démontrée dans la souche de levure RFY 231 (décrite dans Finley Jr, *et al,* 1998, Proc Natl Acad Sci USA, 95, 14266-71). Cette souche de levure possède le génotype (MATα *trp1Δ::hisG his3 ura3-1 leu2::3*Lexop*-LEU2),* et est dérivée de EGY48 (Guris *et al.,* 1993, Cell, 75, 791-803).

Le gène rapporteur était le gène LacZ.

On effectue l'étude sur un milieu contenant du galactose, ne contenant pas de leucine, et on étudie la présence de colonies colorées sur ces boîtes.

### Exemple 7 : criblage de composés interférant avec la liaison Siah - Numb et/ou la quantité de protéine Numb

On effectue les essais décrits dans les exemples 1 à 6, en ajoutant les composés que l'on désire étudier, et on compare avec les résultats obtenus lorsque les composés ne sont pas ajoutés.

## Revendications

1. Procédé d'identification d'un composé inhibant la liaison de Siah à Numb, **caractérisé en ce qu'**il présente les étapes de :
a) mise en contact in vitro dudit composé avec une cellule mammifère exprimant les protéines Siah et Numb ;
b) identification de l'augmentation de la quantité de protéine Numb dans ladite cellule mammifère par rapport à une cellule témoin avec laquelle ledit composé n'a pas été mis en contact.

2. Procédé d'identification d'un composé inhibant la liaison de Siah à Numb, présentant les étapes de :
a) mise en contact dudit composé avec un système permettant la détermination *in vitro* de la liaison entre Siah et Numb ;
b) identification de la diminution et/ou l'inhibition de la liaison entre Siah et Numb.

3. Procédé d'identification d'un composé pour l'augmentation de la répression tumorale et/ou la mort cellulaire (apoptose), **caractérisé en ce qu'**il présente les étapes de :
a) mise en contact in vitro dudit composé avec une cellule mammifère exprimant les protéines Siah et Numb ;
b) identification de la diminution de la quantité de protéine Numb dans ladite cellule mammifère par rapport à une cellule témoin avec laquelle ledit composé n'a pas été mis en contact.

4. Procédé d'identification d'un composé pour la diminution et/ou l'inhibition de la répression tumorale et/ou la mort cellulaire (apoptose), présentant les étapes de:
a) mise en contact de composés avec un système permettant la détermination *in vitro* de la liaison entre Siah et Numb ;
b) identification des composés induisant la diminution et/ou l'inhibition de la liaison entre Siah et Numb ;
c) mise en contact des composés identifiés dans l'étape b) avec une cellule mammifère exprimant les protéines Siah et Numb ;
d) identification de l'augmentation de la quantité de protéine Numb dans ladite cellule mammifère par rapport à une cellule témoin avec laquelle ledit composé n'a pas été mis en contact.

5. Procédé d'identification d'un composé pour l'augmentation de la répression tumorale et/ou la mort cellulaire (apoptose), **caractérisé en ce qu'**il présente les étapes de :
a) identification de composés inhibant la liaison entre Siah et Numb, par un procédé selon la revendication 2 ;
b) mise en oeuvre d'un procédé selon la revendication 3 sur lesdits composés identifiés dans l'étape a).

6. Procédé d'identification d'une région de Siah active pour l'augmentation de la suppression tumorale et/ou de l'apoptose, comportant les étapes de :
a) mise en contact de peptides issus de la protéine Siah avec une cellule mammifère exprimant la protéine Numb ;
b) identification de la diminution de la quantité de protéine Numb dans ladite cellule mammifère par comparaison avec la quantité de protéine Numb présente dans une cellule mammifère témoin exprimant les protéines Siah et Numb.

7. Procédé d'identification d'une région de Siah active pour la diminution et/ou l'inhibition de la suppression tumorale et/ou de l'apoptose, comportant les étapes de :
a) mise en contact in vitro de peptides issus de la protéine Siah avec une cellule mammifère exprimant la protéine Numb ;
b) identification de l'augmentation de la quantité de protéine Numb dans ladite cellule mammifère par comparaison avec la quantité de protéine Numb présente dans une cellule mammifère témoin exprimant les protéines Siah et Numb.

8. Procédé d'identification des régions de Siah impliquées dans la liaison avec la protéine Numb, comportant les étapes de :
a) mise en contact de peptides issus de la protéine Siah dans un système permettant la détermination *in vitro* de la liaison entre Siah et Numb ;
b) identification des peptides entraînant la diminution de la liaison entre. Siah et Numb dans ledit système ;

9. Procédé d'identification d'un produit ayant une activité d'augmentation de la répression tumorale et/ou de l'apoptose, **caractérisé en ce qu'**il comporte les étapes de :
a) mise en oeuvre d'un procédé selon l'une des revendications 3, 5 ou 6,
b) modification du produit sélectionné à l'étape a) notamment par greffage de résidus sur le squelette chimique,
c) test du produit modifié dans l'étape b) dans des procédés *in vitro* et/ou *in vivo,* sur des modèles non-humains pertinents de répression tumorale et/ou d'apoptose,
d) identification du produit permettant d'obtenir une activité de répression tumorale et/ou d'apoptose supérieure à l'activité obtenue pour le produit sélectionné à l'étape a).

10. Procédé d'identification d'un produit ayant une activité de diminution et/ou d'inhibition de la répression tumorale et/ou de l'apoptose, **caractérisé en ce qu'**il comporte les étapes de :
a) mise en oeuvre d'un procédé selon l'une des revendications 1, 2, 4 ou 7,
b) modification du produit sélectionné à l'étape a) notamment par greffage de résidus sur le squelette chimique,
c) test du produit modifié dans l'étape b) dans des procédés *in vitro* et/ou *in vivo,* sur des modèles non-humains pertinents de répression tumorale et/ou d'apoptose,
d) identification du produit permettant d'obtenir une activité de répression tumorale et/ou d'apoptose réduite par rapport à J'activité obtenue pour le produit sélectionné à l'étape a).

11. Procédé selon la revendication 1 ou 4, **caractérisé en ce que** ladite cellule mammifère est transfectée par au moins un vecteur choisi parmi un vecteur portant un fragment d'ADN codant pour Siah, un vecteur portant un fragment d'ADN codant pour Numb, un vecteur portant un fragment d'ADN pour Siah et un fragment d'ADN codant pour Numb.

12. Procédé selon la revendication 1 ou 4, **caractérisé en ce que** l'identification de l'augmentation de la quantité de protéine Numb dans ladite cellule mammifère est effectuée par Western Blot.

13. Complexe constitué d'une protéine Siah et d'une protéine Numb.

## Claims

1. Process for identifying a compound which inhibits the binding of Siah to Numb, **characterized in that** it exhibits the steps of:
a) bringing the said compound into contact in vitro with a mammalian cell which is expressing the Siah and Numb proteins;
b) identifying the increase in the quantity of Numb protein in the said mammalian cell as compared with a control cell with which the said compound has not been brought into contact.

2. Process for identifying a compound which inhibits the binding of Siah to Numb, which process exhibites the steps of:
a) bringing the said compound into contact with a system which enables the binding between Siah and Numb to be determined in vitro;
b) identifying the decrease in and/or inhibition of the binding between Siah and Numb.

3. Process for identifying a compound for increasing tumour suppression and/or cell death (apoptosis), **characterized in that** it exhibits the steps of:
a) bringing the said compound into contact in vitro with a mammalian cell which is expressing the Siah and Numb proteins;
b) identifying the decrease in the quantity of Numb protein in the said mammalian cell as compared with a control cell with which the said compound has not been brought into contact.

4. Process for identifying a compound for decreasing and/or inhibiting tumour suppression and/or cell death (apoptosis), which process exhibits the steps of:
a) bringing compounds into contact with a system which enables the binding between Siah and Numb to be determined in *vitro;*
b) identifying the compounds which induce a decrease in and/or inhibition of the binding between Siah and Numb;
c) bringing the compounds identified in step b) into contact with a mammalian cell which is expressing the Siah and Numb proteins;
d) identifying the increase in the quantity of Numb protein in the said mammalian cell as compared with a control cell with which the said compound has not been brought into contact.

5. Process for identifying a compound for increasing tumour suppression and/or cell death (apoptosis), **characterized in that** it exhibits the steps of:
a) identifying compounds which inhibit the binding between Siah and Numb, using a process in accordance with Claim 2;
b) implementing a process in accordance with Claim 3 on the said compounds identified in step a).

6. Process for identifying a region of Siah which is active in increasing tumour suppression and/or apoptosis, comprising the steps of:
a) bringing peptides derived from the Siah protein into contact in vitro with a mammalian cell which is expressing the Numb protein;
b) identifying the decrease in the quantity of Numb protein in the said mammalian cell by comparison with the quantity of Numb protein which is present in a control mammalian cell which is expressing the Siah and Numb proteins.

7. Process for identifying a region of Siah which is active in decreasing and/or inhibiting tumour suppression and/or apoptosis, comprising the steps of:
a) bringing peptides derived from the Siah protein into contact in vitro with a mammalian cell which is expressing the Numb protein;
b) identifying the increase in the quantity of Numb protein in the said mammalian cell by comparison with the quantity of Numb protein which is present in a control mammalian cell which is expressing the Siah and Numb proteins.

8. Process for identifying the regions of Siah which are involved in the binding with the Numb protein, comprising the steps of:
a) bringing peptides derived from the Siah protein into contact [lacuna] in a system which enables the binding between Siah and Numb to be determined *in vitro;*
b) identifying the peptides which bring about the decrease in the binding between Siah and Numb in the said system.

9. Process for identifying a product which is active in increasing tumour suppression and/or apoptosis, **characterized in that** it comprises the steps of:
a) implementing a process according to one of Claims 3, 5 or 6,
b) modifying the product selected in step a), in particular by attaching residues to the chemical backbone,
c) testing the product modified in step b), in *in vitro* and/or *in vivo* processes, in non-human models which are relevant to tumour suppression and/or apoptosis,
d) identifying the product which makes it possible to obtain a tumour suppression and/or apoptosis activity which is greater than the activity which is obtained in the case of the product selected in step a).

10. Process for identifying a product which is active in decreasing and/or inhibiting tumour suppression and/or apoptosis, **characterized in that** it comprises the steps of:
a) implementing a process according to one of Claims 1, 2, 4 or 7,
b) modifying the product selected in step a), in particular by attaching residues to the chemical backbone,
c) testing the product modified in step b), in *in vitro* and/or *in vivo* processes, in non-human models which are relevant to tumour suppression and/or apoptosis,
d) identifying the product which makes it possible to obtain a tumour suppression and/or apoptosis activity which is reduced as compared with the activity which is obtained in the case of the product selected in step a).

11. Process according to Claim 1 or 4, **characterized in that** the said mammalian cell is transfected with at least one vector which is selected from a vector carrying a DNA fragment encoding Siah, a vector carrying a DNA fragment encoding Numb, and a vector carrying a DNA fragment encoding Siah and a DNA fragment encoding Numb.

12. Process according to Claim 1 or 4, **characterized in that** the increase in the quantity of Numb protein in the said mammalian cell is identified by means of Western blotting.

13. Complex which consists of a Siah protein and a Numb protein.

## Patentansprüche

1. Verfahren zur Identifizierung einer Verbindung, welche die Bindung von Siah an Numb hemmt, **dadurch gekennzeichnet, dass** es die folgenden Schritte aufweist:
a) Inkontaktbringen der Verbindung *in vitro* mit einer Säugetierzelle, welche die Proteine Siah und Numb exprimiert;
b) Identifizierung der Erhöhung der Menge von Numb-Protein in der Säugetierzelle bezogen auf eine Vergleichszelle, mit welcher die Verbindung nicht in Kontakt gebracht worden ist.

2. Verfahren zur Identifizierung einer Verbindung, welche die Bindung von Siah an Numb hemmt, welches die folgenden Schritte aufweist:
a) Inkontaktbringen der Verbindung mit einem System, welches die *in vitro*-Bestimmung der Bindung zwischen Siah und Numb erlaubt;
b) Identifizierung der Verringerung und/oder der Hemmung der Bindung zwischen Siah und Numb.

3. Verfahren zur Identifizierung einer Verbindung für die Erhöhung der Tumorrepression und/oder des Zelltods (Apoptose), **dadurch gekennzeichnet, dass** es die folgenden Schritte aufweist:
a) Inkontaktbringen der Verbindung *in vitro* mit einer Säugetierzelle, welche die Proteine Siah und Numb exprimiert;
b) Identifizierung der Verringerung der Menge von Numb-Protein in der Säugetierzelle bezogen auf eine Vergleichszelle, mit welcher die Verbindung nicht in Kontakt gebracht worden ist.

4. Verfahren zur Identifizierung einer Verbindung für die Verringerung und/oder die Hemmung der Tumorrepression und/oder des Zelltods (Apoptose), **dadurch gekennzeichnet, dass** es die folgenden Schritte aufweist:
a) Inkontaktbringen von Verbindungen mit einem System, welches die *in vitro*-Bestimmung der Bindung zwischen Siah und Numb erlaubt;
b) Identifizierung der Verbindungen, welche die Verringerung und/oder die Hemmung der Bindung zwischen Siah und Numb induzieren;
c) Inkontaktbringen der in Schritt b) identifizierten Verbindungen mit einer Säugetierzelle, welche die Proteine Siah und Numb exprimiert;
d) Identifizierung der Erhöhung der Menge von Numb-Protein in der Säugetierzelle bezogen auf eine Vergleichszelle, mit welcher die Verbindung nicht in Kontakt gebracht worden ist.

5. Verfahren zur Identifizierung einer Verbindung für die Erhöhung der Tumorrepression und/oder des Zelltods (Apoptose), **dadurch gekennzeichnet, dass** es die folgenden Schritte aufweist:
a) Identifizierung von Verbindungen, welche die Bindung zwischen Siah und Numb hemmen, durch ein Verfahren nach Anspruch 2;
b) Ausführen eines Verfahrens nach Anspruch 3 mittels der in dem Schritt a) identifizierten Verbindungen.

6. Verfahren zur Identifizierung einer Region von Siah, die hinsichtlich der Erhöhung der Tumorsuppression und/oder der Apoptose aktiv ist, welches die folgenden Schritte umfasst:
a) Inkontaktbringen von von dem Siah-Protein abstammenden Peptiden *in vitro* mit einer Säugetierzelle, welche das Protein Numb exprimiert;
b) Identifizierung der Verringerung der Menge von Numb-Protein in der Säugetierzelle verglichen mit der Menge von Numb-Protein, welche in einer als Vergleich dienenden Säugetierzelle, welche die Proteine Siah und Numb exprimiert, vorhanden ist.

7. Verfahren zur Identifizierung einer Region von Siah, die hinsichtlich der Verringerung und/oder der Hemmung der Tumorsuppression und/oder der Apoptose aktiv ist, welches die folgenden Schritte umfasst:
a) Inkontaktbringen von von dem Siah-Protein abstammenden Peptiden *in vitro* mit einer Säugetierzelle, welche das Protein Numb exprimiert;
b) Identifizierung der Erhöhung der Menge von Numb-Protein in der Säugetierzelle verglichen mit der Menge von Numb-Protein, welche in einer als Vergleich dienenden Säugetierzelle, welche die Proteine Siah und Numb exprimiert, vorhanden ist.

8. Verfahren zur Identifizierung der Regionen von Siah, welche an der Bindung an das Numb-Protein beteiligt sind, welches die folgenden Schritte umfasst:
a) Inkontaktbringen von von dem Siah-Protein abstammenden Peptiden in einem System, welches die *in vitro*-Bestimmung der Bindung zwischen Siah und Numb erlaubt;
b) Identifizierung der Peptide, welche die Verringerung der Bindung zwischen Siah und Numb in dem System zur Folge haben.

9. Verfahren zur Identifizierung eines Produkts mit einer Aktivität hinsichtlich der Erhöhung der Tumorrepression und/oder der Apoptose, **dadurch gekennzeichnet, dass** es die folgenden Schritte umfasst:
a) Ausführen eines Verfahrens nach einem der Ansprüche 3, 5 oder 6,
b) Modifizierung des in Schritt a) ausgewählten Produkts insbesondere durch Aufpfropfen von Resten auf das chemische Grundgerüst,
c) Test des in Schritt b) modifizierten Produkts in *in vitro-* und/oder *in vivo*-Verfahren mittels sachdienlicher nicht-humaner Modelle von Tumorrepression und/oder Apoptose,
d) Identifizierung des Produkts, welches erlaubt, eine Aktivität hinsichtlich Tumorrepression und/oder Apoptose, welche höher als die für das in Schritt a) ausgewählte Produkt erhaltene Aktivität ist, zu erhalten.

10. Verfahren zur Identifizierung eines Produkts mit einer Aktivität hinsichtlich der Verringerung und/oder Hemmung der Tumorrepression und/oder der Apoptose, **dadurch gekennzeichnet, dass** es die folgenden Schritte umfasst:
a) Ausführen eines Verfahrens nach einem der Ansprüche 1, 2, 4 oder 7,
b) Modifizierung des in Schritt a) ausgewählten Produkts insbesondere durch Aufpfropfen von Resten auf das chemische Grundgerüst,
c) Test des in Schritt b) modifizierten Produkts in *in vitro-* und/oder *in vivo-*Verfahren mittels sachdienlicher nicht-humaner Modelle von Tumorrepression und/oder Apoptose,
d) Identifizierung des Produkts, welches erlaubt, eine Aktivität hinsichtlich Tumorrepression und/oder Apoptose, welche bezogen auf die für das in Schritt a) ausgewählte Produkt erhaltene Aktivität verringert ist, zu erhalten.

11. Verfahren nach Anspruch 1 oder 4, **dadurch gekennzeichnet, dass** die Säugetierzelle transfiziert ist durch wenigstens einen Vektor, welcher unter einem Vektor, welcher ein Siah kodierendes DNA-Fragment trägt, einem Vektor, welcher ein Numb kodierendes DNA-Fragment trägt, einem Vektor, welcher ein Siah kodierendes DNA-Fragment und ein Numb kodierendes DNA-Fragment trägt, ausgewählt wird.

12. Verfahren nach Anspruch 1 oder 4, **dadurch gekennzeichnet, dass** die Identifizierung der Erhöhung der Menge von Numb-Protein in der Säugetierzelle durch Western-Blot ausgeführt wird.

13. Komplex, welcher aus einem Siah-Protein und einem Numb-Protein gebildet wird.
